# EUROPÄISCHE PATENTSCHRIFT

(11) **EP 1 005 647 B1**
(45) Veröffentlichungstag und Bekanntmachung des Hinweises auf die Patenterteilung: **03.03.2004**
(21) Anmeldenummer: 98951178.7
(22) Anmeldetag: 20.08.1998
(51) Int. Cl.: G01N 33/00, G01N 27/12

(54) **SENSOREINHEIT, INSBESONDERE ZUR LUFTGÜTEMESSUNG**
SENSOR UNIT, ESPECIALLY FOR MEASURING AIR QUALITY
ENSEMBLE CAPTEUR, NOTAMMENT POUR LA MESURE DE LA QUALITE DE L'AIR

(30) Priorität: 23.08.1997 DE 19736824
(43) Veröffentlichungstag der Anmeldung: 07.06.2000
(73) Patentinhaber: ROBERT BOSCH GMBH, 70442 Stuttgart (DE)
(72) Erfinder: PETER, Cornelius, D-77815 Bühl (DE)
(86) Internationale Anmeldenummer: PCT/DE1998/002433
(87) Internationale Veröffentlichungsnummer: WO 1999/010738

(56) Entgegenhaltungen:
- EP-A- 0 527 258
- WO-A-93/10441
- US-A- 5 211 053

## Beschreibung

### Stand der Technik

Die Erfindung betrifft eine Sensoreinheit, insbesondere einen Luftgütesensor nach dem Oberbegriff des Anspruchs 1.

Eine solche Sensoreinheit ist aus EP 0 527 258 A1 bekannt.

Derartige Sensoreinheiten sind als sogenannte Luftgüte-Doppelsensoren einsetzbar, mit denen eine Kohlenmonoxyd- und eine Stickoxyd-Messung zur Quantifizierung der Abgasgüte von Kraftfahrzeug-Verbrennungsmotoren durchgeführt werden kann. Bei modernen Verbrennungsmotoren werden eine Vielzahl von Eingangsparametern im Zuge eines elektronischen Motor-Managements verarbeitet. Dies sind beispielsweise die momentane Motor-Umdrehungszahl, die Gaspedalstellung usw.. Eine weitere Kenngröße für das Motor-Management ist die Schadstoffbelastung der bei der Verbrennung entstehenden Abgase. Diese Schadstoffe werden bekannterweise mit Sensoren gemessen, wobei die Daten der Sensoren beim Motor-Management Berücksichtigung finden.

Aus der internationalen Patentanmeldung WO 93/10441 ist ein Verfahren bekannt, das zur Bestimmung von kleinen Kohlenmonoxyd- und Stickoxydmengen in sauerstoffenthaltenden Gasgemischen einen Sensor verwendet, dessen elektrischer Widerstand sich bei einer Temperaturerhöhung verändert. Die Größe der Veränderung ist von der Kohlenmonoxyd-Konzentration im Gasgemisch abhängig. Die Veränderung stellt somit ein Maß für diese Schadstoffe dar. Für jeden Schadstoff wird ein separater Sensor verwendet, dem jeweils zum Durchlaufen des Temperaturbereichs eine elektrische Heizung zugeordnet ist.

### Vorteile der Erfindung

Die erfindungsgemäße Sensoreinheit sieht vor, daß die Trägerelemente U-förmig zu der Trägereinheit zusammengefaßt sind, wobei die zwei Trägerelemente jeweils einen Schenkel des U bilden und der dazwischenliegende Schlitz die Wärmesperre verkörpert. Ein gemeinsamer Trägerbereich verbindet die Trägerelemente und trägt elektrische Leiterbahnen und Kontakte, die es ermöglichen, die Sensoreinheit in einem elektrischen Aufnahmesockel anzuschließen.

Die Wärmesperre verhindert ein thermisches "Übersprechen" zwischen den Trägerelementen, so daß die erste Wärmequelle nur mit dem ersten Sensorelement und die zweite Wärmequelle nur mit dem zweiten Sensorelement zusammenwirkt, d.h., die Wärmesperre bildet eine thermische Entkoppelung zwischen den beiden Sensorelementen beziehungsweise zwischen dem jeweiligen Sensorelement und diesem Sensorelement nicht zugeordneten Wärmequelle. Durch die erfindungsgemäße Anordnung wird auch die Zeit verkürzt, die die Sensoreinheit nach dem Einschalten der Wärmequelle(n) benötigt, um eine stationäre Temperaturverteilung zu erreichen und genaue Meßergebnisse zu liefern.

Nach einer Weiterbildung der Erfindung ist vorgesehen, daß die beiden Trägerelemente eine einstückige Trägereinheit bilden. Die zuvor benannte "gemeinsame" Trägereinheit muß demzufolge nicht zwangsläufig einstückig sein, sondern kann auch aus zwei Teilen bestehen, die jedoch mit geeigneten Mitteln aneinander befestigt sind. Alternativ ist es jedoch auch möglich - wie oben stehend bereits angegeben, die beiden Trägerelemente als einstückige Trägereinheit zusammenzufassen.

Ferner ist es vorteilhaft, wenn die Trägereinheit als mit elektrischen Leiterbahnen versehenes Substrat ausgebildet ist. Demzufolge bildet die Trägereinheit vorzugsweise eine Leiterplatte.

Die Wärmesperre, die zwischen den beiden Trägerelementen liegt, ist derart zu verstehen, daß sie die Trägereinheit durchsetzt, so daß an dieser Stelle quasi eine thermische Luftisolationsstelle gebildet wird. Sie kann mehrere reihenförmig angeordnete Ausnehmungen umfassen, zwischen denen lediglich dünne Materialstege der Trägereinheit liegen, wobei diese dünnen Materialstege zur gewünschten thermischen Entkoppelung beitragen. Die erwähnte Ausnehmung erstreckt sich nicht über die gesamte Längserstreckung der Trägereinheit, sondern nur über einen Teilabschnitt der Trägereinheit, so daß rechts und links davon die Heizungen und auch die Sensorelemente liegen und im aussparungsfreien Bereich, dem gemeinsamen Trägerbereich die elektrischen Zuleitungen und Kontakte liegen, um die Sensoreinheit in einem elektrischen Aufnahmesockel einstecken zu können.

Nach einer Weiterbildung der Erfindung ist vorgesehen, daß die beiden Wärmequellen als elektrische Widerstandsleitungen ausgebildet sind. Die beiden Widerstandsleitungen können als Widerstandsleiterbahnen auf dem Substrat ausgebildet sein. Dies führt zu einer sehr einfachen Bauform.

Ferner ist es vorteilhaft, wenn die beiden Sensorelemente von auf dem Substrat angeordneten Sensorleiterbahnen gebildet sind.

Wird als Substrat eine mit Leiterbahnen versehene Leiterplatte verwendet, so ließt ein plattenförmiges Substrat vor, wobei sich die Widerstandsleiterbahnen auf der einen Seite und die Sensorleiterbahnen auf der anderen Seite des Substrats befinden. Demzufolge liegt jeweils eine Widerstandsleiterbahn und eine Sensorleiterbahn auf Vorder- und Rückseite des Substrats, wobei zwischen den beiden Widerstandsbahnen bzw. zwischen den beiden Sensorleiterbahnen die Wärmesperre, insbesondere in Form eines Schlitzes, liegt.

Die Leiterbahnen sind vorzugsweise bis in den Randbereich des Substrats geführt und bilden dort Steckkontakte und/oder Löt- bzw. Schweißkontakte.

Ferner ist es vorteilhaft, wenn die beiden Widerstandsleiterbahnen in Reihe geschaltet sind, da dann die Anzahl der Steckkontakte bzw. Löt- oder Schweißkontakte sehr gering ist.

Nach einer Weiterbildung der Erfindung ist vorgesehen, daß mindestens eine Widerstandsleiterbahn mit mindestens einer Sensorleiterbahn mittels einer Durchkontaktierung durch das Substrat elektrisch leitfähig miteinander verbunden sind. Die Durchkontaktierung ist bevorzugt mit nur einem Steckkontakt und/oder Löt- bzw. Schweißkontakt elektrisch verbunden, so daß die Durchkontaktierung ein gemeinsames elektrisches Potential, vorzugsweise das Massepotential, den elektrischen Heizungen und den beiden Sensoreinheiten zuführt.

Die Zeichnung veranschaulicht die Erfindung anhand eines Ausführungsbeispiels und zwar zeigt:
- Figur 1: eine Frontansicht einer Meß- bzw. Sensorseite einer Sensoreinheit und
- Figur 2: eine Rückansicht einer Heizseite der Sensoreinheit.

Das Ausführungsbeispiel beschreibt eine Sensoreinheit 2 zur Messung der Abgasbelastung eines Verbrennungsmotors, speziell zur Bestimmung der Stickoxyd- und Kohlenmonoxydkonzentration. Die Sensoreinheit 2 weist eine Leiterplatte 21 auf, die durch eine Ober- und Unterkante 22, 23 und zwei Seitenkanten 24 begrenzt wird. Auf der Leiterplatte 21 sind beidseitig Leiterbahnen 25 aufgebracht. Die Leiterplatte 21 weist auf ihrer Vorderseite zwei Sensorelemente 3, 4 auf. Die Sensorelemente beanspruchen in ihrer Längserstreckung, von der Oberkante 22 beginnend, etwa dreiviertel der Länge zwischen Oberkante 22 und Unterkante 23. Im Bereich der Unterkante 23 befinden sich die Anschlüsse der beiden Sensorelemente 3, 4. Die Leiterplatte 21 weist auf ihrer Rückseite zwei Heizelemente 26, 27 auf. Die Heizelemente 26, 27 erwärmen im Betrieb die auf der Vorderseite der Leiterbahn 21 angebrachten Sensorelemente 3, 4 um die Messung der Abgasbelastung durchführen zu können.

Im folgenden wird der konstruktive Aufbau der Sensoreinheit 2 näher erläutert.

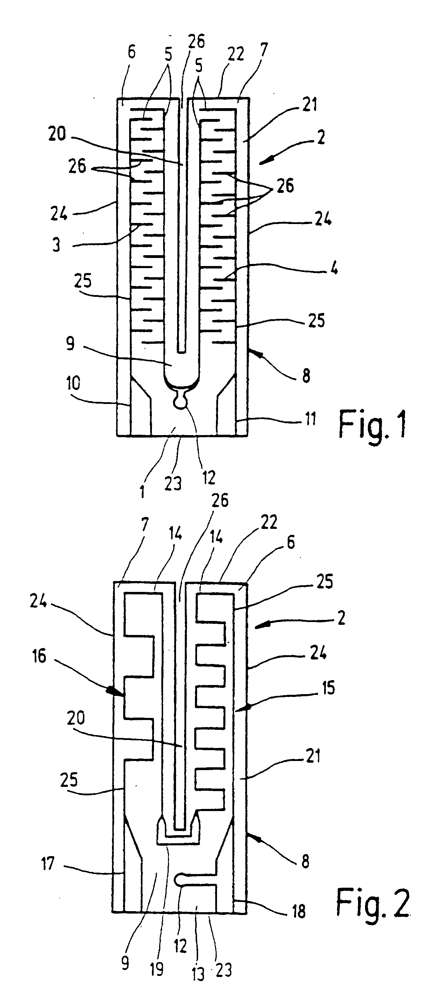

Figur 1 zeigt die Vorderseite der Leiterplatte 21, Frontansicht einer Sensorseite 1. Im folgenden wird die Vorderseite als eine Sensorseite 1 bezeichnet. Durch eine mittige Ausnehmung 20 in einem, in Längsrichtung der Sensoreinheit parallel zu den Seitenkanten angeordneten Teilbereich der Sensoreinheit 2, nimmt die Sensoreinheit 2 eine U-förmige Gestalt an. Auf der Sensorseite 1 ist ein erstes Sensorelement 3 und ein zweites Sensorelement 4 in Form von Sensorleiterbahnen 5 aufgebracht. Die Sensoreinheit 2 besteht aus einem ersten Trägerelement 6, dem durch die Ausnehmung 20, ein zweites Trägerelement 7 gegenübersteht. Das erste Trägerelement 6, das zweite Trägerelement 7 und ein gemeinsamer Trägerbereich 9 bilden zusammen eine Trägereinheit 8.

Die Sensorleiterbahn 5 ist von einer verbreitert ausgebildeten ersten Sensorkontaktierung 10, die sich im Bereich der Unterkante 23 befindet, parallel an der Seitenkante 24 des ersten Trägerelements 6 entlang bis in den Bereich der Oberkante 22 des ersten Trägerelements 6 aufgebracht. Entsprechend ist für die Bildung des zweiten Sensorelements 4, die Sensorleiterbahn 5 gleichartig von einer verbreitert ausgebildeten zweiten Sensorkontaktierung 11 auf das zweite Trägerelement 7 geführt.

Von der geradlinig in die beiden Trägerelemente 6, 7 geführten Sensorleiterbahn 5 zweigen kurze Stege 26 rechtwinklig in Richtung der Ausnehmung 20 ab, so daß eine kammförmigen Sensorleiterbahn 5 gebildet wird. Dieser kammförmigen Sensorleiterbahn 5 stehen jeweils auf beiden Trägerelementen 6, 7 ebenso kammförmig ausgebildete Sensorleiterbahnen 5 versetzt gegenüber, so daß die Stege der äußeren Sensorleiterbahn 5 in die Lücken zwischen den Stegen 26 der inneren Sensorleiterbahnen 5 greifen und umgekehrt. Zwischen den inneren Sensorleiterbahnen 5 und den äußeren Sensorleiterbahnen 5 bestehen keine elektrischen Verbindungen.

Im gemeinsamen Trägerbereich 9 ist eine Durchkontaktierung 12 vorgesehen, um eine gemeinsame Masse von Sensorseite 1 auf die, auf der Rückseite liegende, Heizseite 13 zu führen. Die beiden kammförmig ausgebildeten innenliegenden Sensorleiterbahnen 5 sind zu der Durchkontaktierung 12 geführt.

In Figur 2 ist die Rückseite, der in Figur 1 dargestellten Sensoreinheit 2, dargestellt. Die Figur 2 stellt somit die Heizseite 13 dar. Auf beiden Trägerelementen 6, 7 ist eine durchgängige Widerstandsleiterbahn 14 aufgebracht. Die Widerstandsleiterbahn 14 ist von einer verbreiterten Heizbahnkontaktierung 17, im Bereich der Unterkante 23, mäanderförmig auf das zweite Trägerelement 7 geführt und läuft geradlinig, parallel zur Seitenkante 24 der Sensoreinheit 2 zurück zu einem verbreiterten Übergangsbereich 19. Von dem übergangsbereich 19 verläuft die Widerstandsbahn 14 wieder mäanderförmig auf dem ersten Trägerelement 6 hoch zur Oberkante 22, und von dort aus geradlinig, parallel zur Seitenkante 24 der Sensoreinheit 2, zurück zu einer verbreiterten Massekontaktierung 18. Das Ausführungsbeispiel zeigt hier auf dem ersten Trägerelement 6 engere Leitungsschleifen, als auf dem zweiten Trägerelement 7. Damit ergeben sich verschiedene Längenabschnitte der Widerstandsleiterbahn 14 und somit können beide Trägerelemente 6, 7 mit unterschiedlichen, und damit für die Luftgütemessung notwendigen, Temperaturen beaufschlagt werden. Aus dieser Anordnung der Widerstandsleiterbahn 14 auf den beiden Trägerelementen 6 und 7 ergibt sich eine Reihenschaltung einer ersten Wärmequelle 15, des ersten Trägerelements 6 und einer zweiten Wärmequelle 16, des zweiten Trägerelements 7. Von der Massekontaktierung 18 führt, auf dem gemeinsamen Trägerbereich 9, eine verbreiterte Leiterbahn zur Durchkontaktierung 12. In dem Übergangsbereich 19, zwischen den beiden Wärmequellen 15, 16 ist die Widerstandsleiterbahn 14 in ihrer horizontalen Ausdehnung verbreitert, um damit in dem gemeinsamen Trägerbereich 9 einen geringeren elektrischen widerstand, als im zu heizenden Bereich, zu erreichen und somit eine geringere Wärmeabstrahlung hervorzurufen.

Figur 1 und Figur 2 zeigen deutlich zwischen den beiden Trägerelementen 6, 7 in der einstückigen Sensoreinheit 2 die Ausnehmung 20 in Form eines geradlinigen Schlitzes 26, um damit eine größere thermische Entkoppelung zwischen den beiden Trägerelementen zu erhalten.

In einer weiteren, hier nicht dargestellten, erfindungsgemäßen Ausführungsform können die beiden Sensorkontaktierungen 10, 11 auch in einer anderen, nicht wie in Figur 1 dargestellten Art und Weise, ausgebildet sein; so ist es zum Beispiel denkbar, daß die verbreiterten Sensorleiterbahnen 5 nicht bis an die Unterkante 23 der Trägereinheit 8 geführt sind und damit die beiden Sensorkontaktierungen 10, 11 auch nicht bis an die Unterkante 23 der Trägereinheit 8 geführt sind.

Eine Halterung der Sensoreinheit 2, eine Kontaktiervorrichtung und eine diese ansteuernde Meß- und Steuerungselektronik mitsamt einem gemeinsamen Gehäuse ist in den Figuren 1 und 2 aus Gründen der Übersicht nicht dargestellt.

### Funktionsweise der Sensoreinheit

Durch Anlegen einer Heizspannung zwischen der Heizbahnkontaktierung 17 und der Massekontaktierung 18 fließt ein Heizstrom durch die Widerstandsleiterbahn 14. Durch die geeignete Wahl von Widerstandsleiterbahn-Material, -dicke und deren Führung wird, hervorgerufen durch den Stromfluß, mit den beiden Wärmequellen 15, 16, die beiden jeweils auf der Vorderseite der Sensoreinheit 2 liegenden Sensorelemente 3, 4 beheizt. Durch eine Spannungsänderung und der damit resultierenden Stromänderung kann somit eine Temperaturänderung durchgeführt werden und damit ein charakteristischer Temperaturbereich durchlaufen werden.

Durch Anlegen zweier Meßspannungen zwischen der ersten bzw. der zweiten Sensorkontaktierung 10, 11 und der gemeinsamen Massekontaktierung 18 auf der Heizseite 13 wird mittels einer nicht dargestellten Meß- und Steuerelektronik und dem Durchlaufen eines für die jeweiligen Sensoreinheiten 2, 3, charakteristischen Temperaturbereichs der jeweilige zugehörige elektrische Widerstand gemessen. Die auf diese Weise ermittelten Kennlinien (Abhängigkeit von Temperatur und elektrischen Widerstand) sind durch Abweichung von einer Referenzkennlinie für unbelastete Luft, ein Maß für die Stickoxyd- bzw. Kohlenmonoxydbelastung der Luft. Somit ist eine Bestimmung der Luftgüte durch Messung zweier, der unter anderem für die Luftbelastung relevanten chemischen Verbindungen, Stickoxyd und Kohlenmonoxyd, durch die Verwendung einer einstückigen bzw. einteiligen Sensoreinheit, die sich durch einen einfachen Aufbau und der damit verbundenen Material- und Herstellungskosten auszeichnet, durchführbar.

## Patentansprüche

1. Sensoreinheit, insbesondere Luftgütesensor, mit einer Trägereinheit (8), die wenigstens zwei Trägerelemente (6,7) umfaßt, auf denen jeweils ein Sensorelement und eine diesem zugeordnete Wärmequelle angeordnet sind, und die zur thermischen Entkopplung zwischen sich eine Wärmesperre aufweisen, **dadurch gekennzeichnet, daß** die Trägerelemente (6,7) U-förmig zu der Trägereinheit (8) zusammengefaßt sind, wobei die zwei Trägerelemente (6,7) jeweils einen Schenkel des U bilden und der Schlitz (26) zwischen den zwei Schenkeln die Wärmesperre darstellt, und daß ein gemeinsamer Trägerbereich (9) die Trägerelemente (6,7) miteinander verbindet und elektrische Leiterbahnen und Kontakte (10,11) trägt, um die Sensoreinheit in einem elektrischen Aufnahmesockel anschließen zu können.

2. Sensoreinheit nach Anspruch 1, **dadurch gekennzeichnet, daß** die Trägereinheit einstückig ist.

3. Sensoreinheit nach einem der vorherigen Ansprüche, **dadurch gekennzeichnet, daß** die Trägereinheit (8) als mit elektrischen Leiterbahnen (25) versehenes Substrat ausgebildet ist.

4. Sensoreinheit nach einem der vorherigen Ansprüche, **dadurch gekennzeichnet, daß** die beiden Wärmequellen als elektrische Widerstandsleitungen ausgebildet sind.

5. Sensoreinheit nach Anspruch 4, **dadurch gekennzeichnet, daß** die beiden Widerstandsleitungen Widerstandsleiterbahnen (14) auf dem Substrat aufweisen.

6. Sensoreinheit nach einem der vorherigen Ansprüche, **dadurch gekennzeichnet, daß** die beiden Sensorelemente (3,4) von auf dem Substrat angeordneten Sensorleiterbahnen (5) gebildet sind.

7. Sensoreinheit nach Anspruch 6, **dadurch gekennzeichnet, daß** das Substrat plattenförmig ausgebildet ist und daß sich die Widerstandsleiterbahnen (14) auf der einen Seite und sich die Sensorleiterbahnen (5) auf der anderen Seite des Substrats befinden.

8. Sensoreinheit nach einem der vorherigen Ansprüche, **dadurch gekennzeichnet, daß** die Leiterbahnen (25) bis in den Randbereich des Substrats geführt sind und dort Steckkontakte (11,12) und/oder Lötbeziehungsweise Schweißkontakte bilden.

9. Sensoreinheit nach Anspruch 4 oder 5, **dadurch gekennzeichnet, daß** die beiden Widerstandsleiterbahnen (14) in Reihe geschaltet sind.

10. Sensoreinheit nach einem der vorherigen Ansprüche, **dadurch gekennzeichnet, daß** mindestens eine Widerstandsleiterbahn (14) mit mindestens einer Sensorleiterbahn (5) mittels einer Durchkontaktierung (12) durch das Substrat elektrisch leitfähig miteinander verbunden sind.

11. Sensoreinheit nach Anspruch 10, **dadurch gekennzeichnet, daß** die Durchkontaktierung (12) mit nur einem Steckkontakt und/oder Löt- beziehungsweise Schweißkontakt elektrisch verbunden ist.

## Claims

1. Sensor unit, in particular air quality sensor, having a carrier unit (8), which comprises at least two carrier elements (6, 7), on each of which a sensor element and a heat source assigned thereto are arranged, and which have a heat barrier for thermal decoupling between them, **characterized in that** the carrier elements (6, 7) are combined in U-shaped fashion to form the carrier unit (8), the two carrier elements (6, 7) each forming a limb of the U and the slot (26) between the two limbs representing the heat barrier, and **in that** a common carrier region (9) connects the carrier elements (6, 7) to one another and carries electrical conductor tracks and contacts (10, 11) in order to be able to connect the sensor unit in an electrical receptacle base.

2. Sensor unit according to Claim 1, **characterized in that** the carrier unit is in one piece.

3. Sensor unit according to one of the preceding claims, **characterized in that** the carrier unit (8) is formed as a substrate provided with electrical conductor tracks (25).

4. Sensor unit according to one of the preceding claims, **characterized in that** the two heat sources are formed as electrical resistance lines.

5. Sensor unit according to Claim 4, **characterized in that** the two resistance lines have resistance conductor tracks (14) on the substrate.

6. Sensor unit according to one of the preceding claims, **characterized in that** the two sensor elements (3, 4) are formed by sensor conductor tracks (5) arranged on the substrate.

7. Sensor unit according to Claim 6, **characterized in that** the substrate is embodied in plate form, and **in that** the resistance conductor tracks (14) are situated one side and the sensor conductor tracks (5) are situated on the other side of the substrate.

8. Sensor unit according to one of the preceding claims, **characterized in that** the conductor tracks (25) are led right into the edge region of the substrate and there form plug contacts (11, 12) and/or soldering or welding contacts.

9. Sensor unit according to Claim 4 or 5, **characterized in that** the two resistance conductor tracks (14) are connected in series.

10. Sensor unit according to one of the preceding claims, **characterized in that** at least one resistance conductor track (14) with at least one sensor conductor track (5) are electrically conductively connected to one another by means of a plated-through hole (12) through the substrate.

11. Sensor unit according to Claim 10, **characterized in that** the plated-through hole (12) is electrically connected to only one plug contact and/or soldering or welding contact.

## Revendications

1. Ensemble capteur, notamment capteur de la qualité de l'air, comportant un ensemble de support (8) qui comprend au moins deux éléments supports (6, 7), sur lesquels un élément capteur et une source de chaleur associée à celui-ci sont disposés respectivement et qui présentent une barrière thermique pour le découplage thermique entre eux,
**caractérisé en ce que**
les éléments supports (6, 7) sont assemblés en forme de U au niveau de l'unité de support (8), les deux éléments supports (6, 7) formant chacun une branche du U et la fente (26) entre les deux branches représentant la barrière thermique, et une zone support commune (9) relie les éléments supports (6, 7) l'un avec l'autre et supporte des conducteurs et contacts électriques (10, 11), afin de pouvoir raccorder l'ensemble capteur dans un socle électrique de réception.

2. Ensemble capteur selon la revendication 1,
**caractérisé en ce que**
l'ensemble support est en un bloc.

3. Ensemble capteur selon l'une quelconque des revendications précédentes,
**caractérisé en ce que**
l'ensemble support (8) est conçu en tant que substrat muni de conducteurs électriques (25).

4. Ensemble capteur selon l'une quelconque des revendications précédentes,
**caractérisé en ce que**
les deux sources de chaleur sont conçues en tant que fils électriques constituant une résistance.

5. Ensemble capteur selon la revendication 4,
**caractérisé en ce que**
les deux fils de résistance présentent des conducteurs de résistance (14) sur le substrat.

6. Ensemble capteur selon l'une quelconque des revendications précédentes,
**caractérisé en ce que**
les deux éléments capteurs (3, 4) sont formés par les conducteurs de capteur (5) disposés sur le substrat.

7. Ensemble capteur selon la revendication 6,
**caractérisé en ce que**
le substrat est conçu en forme de plaque, les conducteurs de résistance (14) se trouvent d'un côté et les conducteurs de capteur (5) de l'autre côté du substrat.

8. Ensemble capteur selon l'une quelconque des revendications précédentes,
**caractérisé en ce que**
les conducteurs (25) sont amenés jusqu'à la zone du bord du substrat et forment là des contacts à fiche (11, 12) et/ou des contacts à braser, ou à souder.

9. Ensemble capteur selon la revendication 4 ou 5,
**caractérisé en ce que**
les deux conducteurs de résistance (14) sont montés en série.

10. Ensemble capteur selon l'une quelconque des revendications précédentes,
**caractérisé en ce qu'**
au moins un conducteur de résistance (14) est relié, de manière à conduire l'électricité, avec au moins un conducteur de capteur (5) au moyen d'une mise en contact (12) par le biais du substrat.

11. Ensemble capteur selon la revendication 10,
**caractérisé en ce que**
la mise en contact (12) est reliée électriquement avec seulement un contact à fiche et/ou un contact à braser ou à souder.
